# EUROPEAN PATENT SPECIFICATION

(11) **EP 3 363 474 B1**
(45) Date of publication and mention of the grant of the patent: **30.09.2020**
(21) Application number: 18157765.1
(22) Date of filing: 21.02.2018
(51) Int. Cl.: A61L 2/24, A61L 2/26, A61B 50/33

(54) **MODULAR TRAY SUPPORT FOR AUTOCLAVE**
MODULARER SCHALENTRÄGER FÜR AUTOKLAVE
SUPPORT DE PLATEAU MODULAIRE POUR AUTOCLAVE

(30) Priority: 21.02.2017 IT 201700019118
(43) Date of publication of application: 22.08.2018
(73) Proprietor: CEFLA SOCIETA' COOPERATIVA, 40026 Imola (BO) (IT)
(72) Inventor: PAZZI, Juri, 40026 Imola (BO) (IT)
(74) Representative: Karaghiosoff, Giorgio Alessandro

(56) References cited:
- DE-A1- 3 443 912
- US-A1- 2012 087 844
- US-A1- 2012 139 399

## Description

The present invention relates to the technical field of autoclaves for sterilizing surgical/dental/veterinary instruments. More particularly, the present invention relates to a support system, called modular tray support, for the support of cassettes/trays containing the instruments to be sterilized, said modular tray support being placed inside autoclave's sterilization chamber.

In the art, substantially two kinds of autoclaves are known:
- Autoclaves using water steam;
- Autoclaves using chemical vapours, like e.g. alcohols, formaldehyde, ketones, ethylene oxide, and/or mixtures thereof.

The present invention can be used in any kind of counter autoclave, or small autoclave, commonly found in all medical/dental/veterinary practices making use of instruments that must be sterilized after their use.

In the art, inserting instruments to be sterilized in cassettes/trays is common practice, inside/on which said instruments are then stored up to the moment of use. E.g. EP116515 0B1 of Scican describes cassettes of this kind.

A document describing a support system for such a cassette is e.g. EP1175231B1 in the name of Scican again.

In the art, preparing cassettes/trays containing all the instruments needed for a certain kind of therapy is known: e.g., in the non-limiting case of a dental practice, a cassette for orthodontic, conservative, endodontic therapy may be prepared. The difference between a tray and a cassette lies in the fact that the tray is open, while the cassette is closed.

Said sterilized cassette is opened at the moment of performing said therapy on a specific patient, at the end of which all instruments are cleaned and placed in said cassette in order to sterilize them in autoclave, be it a water steam autoclave or a chemical vapour autoclave.

It is worth noting that the presence of a support for cassettes/trays inside autoclave's sterilization chamber is essential to allow the correct circulation of sterilizing fluid inside said chamber. In particular, empty space must be provided between two cassettes, which cannot be simply superimposed. It is apparent that two trays cannot be superimposed, but a support maintaining them at a given distance is needed.

US2012/0087844 discloses a tray support for an autoclave.

Said cassettes, having a parallelepiped shape, have different dimension, according to their content. As a consequence, the loading of autoclave's sterilization chamber becomes problematic when the supporting brackets of cassettes/trays are fixed. In fact, a fixed distance between brackets can make the loading of a very thick cassette more difficult. Moreover, oftentimes said sterilization chamber has a cylindrical shape, while cassettes/trays typically have a parallelepiped shape.

The difficulty in loading a plurality of cassettes/trays inside said sterilization chambers leads to the possibility of loading, e.g., only two cassettes/trays having given dimensions: if the cassettes/trays to be sterilized are three, two successive sterilization cycles must be performed, with ensuing waste of time and energy.

Aim of the present invention is providing an apparatus and a method to allow the maximization of the number of cassettes/trays that can be loaded in an autoclave's sterilization chamber for each sterilization cycle.

This object is achieved by an apparatus and a method having the features of the independent claims. Advantageous embodiment and refinements are specified in the claims dependent thereon.

Substantially, the solution consists in providing a cassettes/trays support system for the sterilization chamber that can be modified according to need, i.e. according to the dimensions of the cassettes/trays to be sterilized, in an easy and speed way, by the human operator loading the autoclave, each time a sterilization cycle must be performed.

Advantageously, the external walls of said support system according to the instant invention have a shape of an annular frame which can show different shapes such as polygonal shapes, rounded shapes or which can be formed by a combination of arched or curved elements and rectilinear elements.

Each frame of the at least three coaxial frames aligned one after the other shows at least two opposite lateral elements and at least one upper and one bottom element.

In an embodiment the frames have a barrel shape, with two parallel opposed walls and the other two opposed curved walls. This shape of the frames is innovative with respect to the traditional hexagonal shape. This allow to better use the available space of sterilization chamber, which has typically a cylindrical shape and as will be described in the following this shape allows also to rotate the tray support of 90° inside the autoclave allowing to obtain a different configuration of the supports for the trays..

The advantages of the present invention are manifold.

A first advantage is the maximization of the cassettes/trays load, with ensuing saving in the number of sterilizing cycles, which translates into a saving of time and energy.

A second advantage is the possibility, for the human operator loading said autoclave, to modify said support system.

A third advantage is the fact that said supporting system provides loading possibilities which can be varied over time, even according to cassettes/trays of new dimensions, not yet available on the market today .

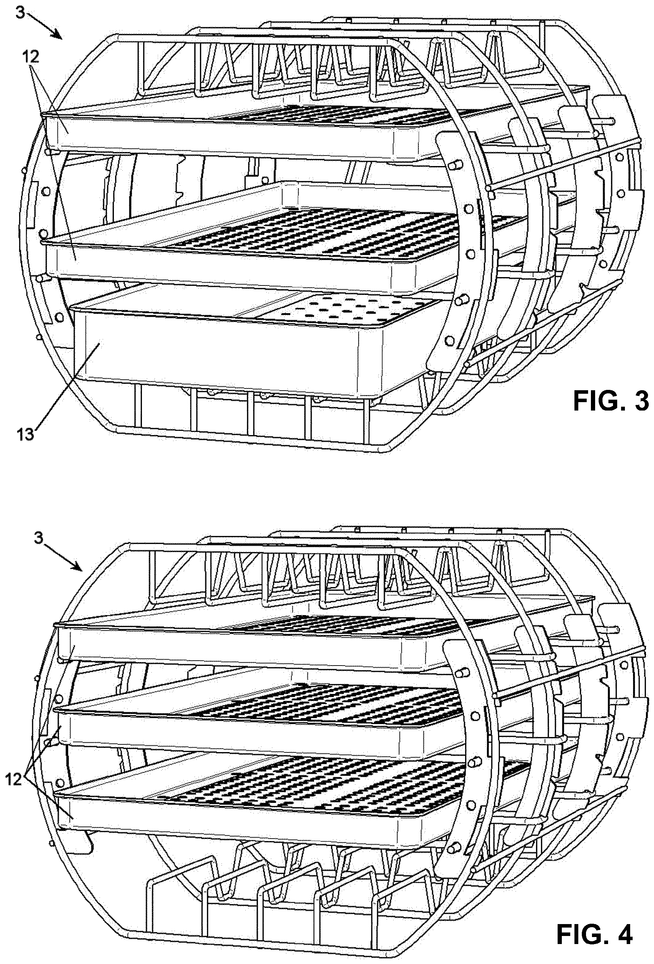

A fourth advantage lies in the possibility of mixed loads of trays/cassettes, as shown in Figure 3, with ensuing saving of time and energy.

Further advantages and properties of the present invention are disclosed in the following description, in which exemplary embodiments of the present invention are explained in detail based on the drawings:
- Figure 1: Axonometric view of a support system inside an autoclave's sterilization chamber;
- Figure 2: Axonometric view of a modular tray support according to the present invention, with all its removable brackets;
- Figure 3: Axonometric view of a configuration of said tray support according to the present invention, with a mixed load comprising two sterilization trays and one cassette, having different dimensions;
- Figure 4: Axonometric view of an alternative configuration of said tray support according to the present invention with a load of three sterilization trays having the same dimensions;
- Figure 5: Axonometric view of said modular tray support skeleton freed from its removable brackets;
- Figure 6: Axonometric view of a bracket fixed to the posterior area, first assembling step;
- Figure 7: Axonometric view of the anterior fixing of said bracket, second assembling step;
- Figure 8: Axonometric view of the blocking of said bracket in its working position, third assembling step;
- Figure 9: Axonometric view of said modular tray support, rotated 90 degrees to support the standard set of sterilizing trays;
- Figure 10: Axonometric view of the modular tray according to the present invention in an embodiment with polygonal sides.

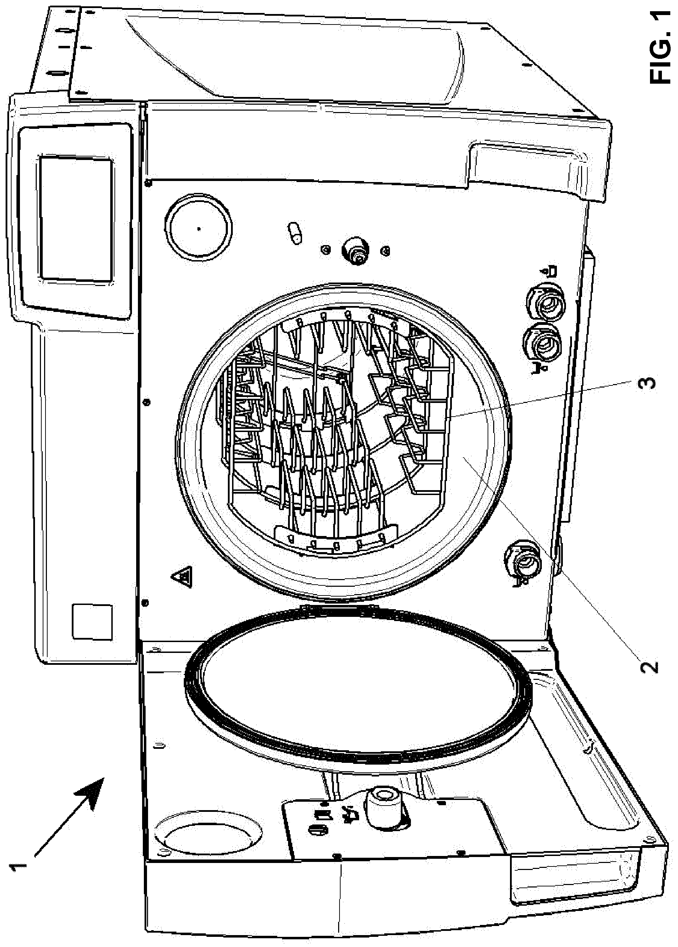

Figure 1 shows an autoclave 1 provided with a sterilization chamber 2 housing a modular tray support 3 according to the present invention. Said sterilization chamber 2 has a cylindrical shape, which can be loaded with different sterilization cassettes/trays (not shown in the present Figure).

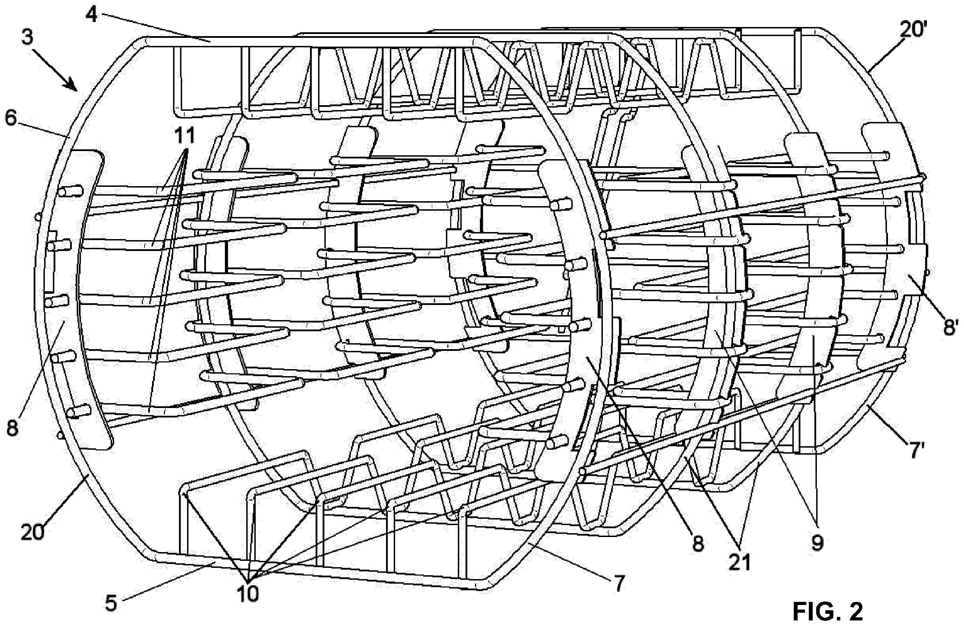

Figure 2 shows an axonometric view of said modular tray support according to the present invention. Said tray support 3 has a metallic electrowelded skeleton, suitable to the cylindrical shape and dimension of said sterilization chamber 2.

Said tray support 3 is a tubular frame comprising a plurality of rings 20, 21 placed parallel to each other, kept at a regular distance by longitudinal elements 10 having a zigzag shape, partially protruding toward the lumen of sterilization chamber 2. Said longitudinal elements 10 are fixed, and their position can never be varied.

In the embodiment shown in Figure 2, four rings are shown: two extremity rings 20 and 20' and two intermediate rings 21; the number of intermediate rings 21 varies according to the dimensions (depth) of said sterilization chamber 2.

Each of said rings 20, 21 has two opposed planar sides 4, 5 and two opposed curved sides 6, 7.

On the internal side of each curved sides 6, 7 of extremity rings 20, two bracket supports 8 are fixed. On the other hand, on said intermediate rings 21 bracket blocks 9 slightly protruding toward the outside with respect to intermediate rings 21 are placed. All said bracket supports 8 and bracket blocks 9 are welded to their respective ring 20, 21.

Figure 3 shows one possible configuration for using said modular tray support 3 according to the present invention: removable brackets 11 support two sterilization trays 12 and one cassette 13 having different dimensions and shapes.

Figure 4 shows a second, alternative configuration for using said modular tray support 3 according to the present invention: said removable brackets 11 support three sterilization trays.

It is apparent that, according to cassette dimensions, they can be placed in different position and numbers. Consequently, removable brackets 11 assembled in said tray support 3, which are all identical, can vary in number and position.

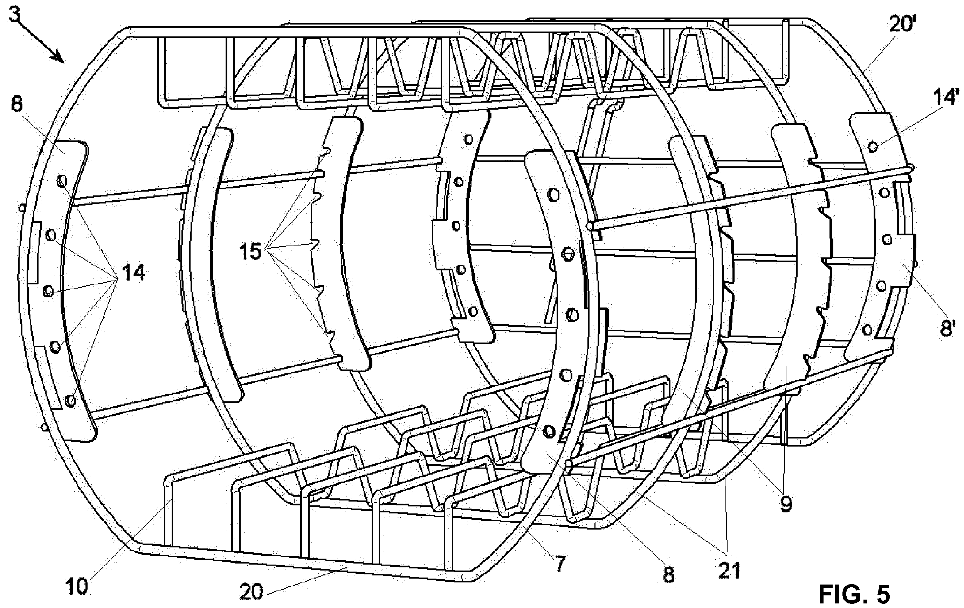

Figure 5 shows said tray support 3 freed from all removable brackets 11 (skeleton).

In Figure 5, said extremity rings 20, 20' are provided, on their respective bracket blocks 8, 8', with a plurality of holes 14, 14' for inserting the ends of removable brackets 11, while intermediate rings 21 have recesses 15 for blocking said removable brackets 11. Said removable brackets 11, too, have a zigzag shape, partially protruding toward the lumen of sterilizing chamber 2.

Brackets 11 are removably fixed to brackets supports 8, and kept in their position by bracket blocks 9.

It is worthwhile noting that all brackets 11 are removable, and removing the non-used ones in a sterilization cycle is preferable.

It is worthwhile noting that, for better convenience, said modular tray support 3 is completely extracted from said sterilization chamber 2 for assembling/disassembling removable brackets 11 according to need. This allows to ease the assembling/disassembling, in that said removable brackets can be fixed working on their outside. Nonetheless, the assembling may be performed also while keeping the skeleton inside sterilization chamber 2.

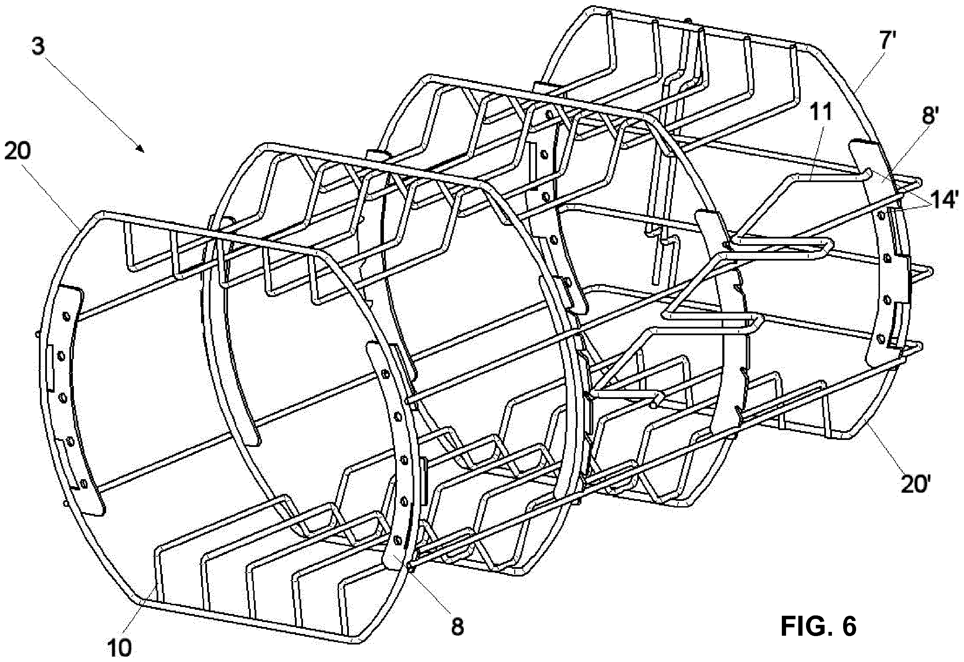

The assembly of modular tray support 3 comprise the following steps:
- Optional extraction of modular tray support 3 from sterilization chamber 2 (Figure 5);
- Insertion of a first end of a removable bracket 11 into a first hole 14'obtained in bracket support 8'fixed to a first extremity ring 20'on a first curved side 7' (Figure 6);

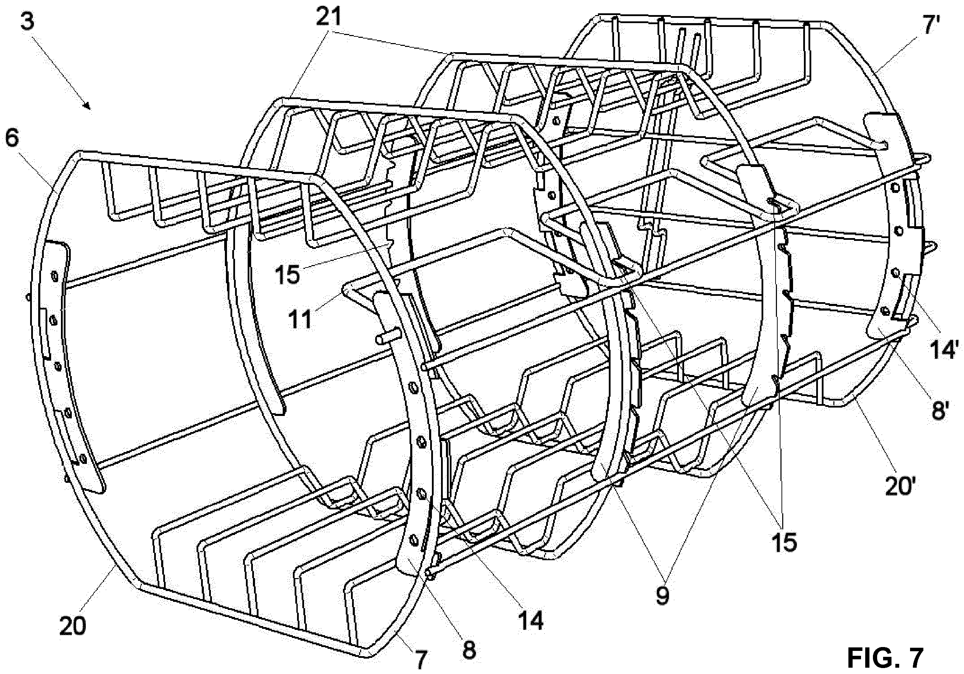

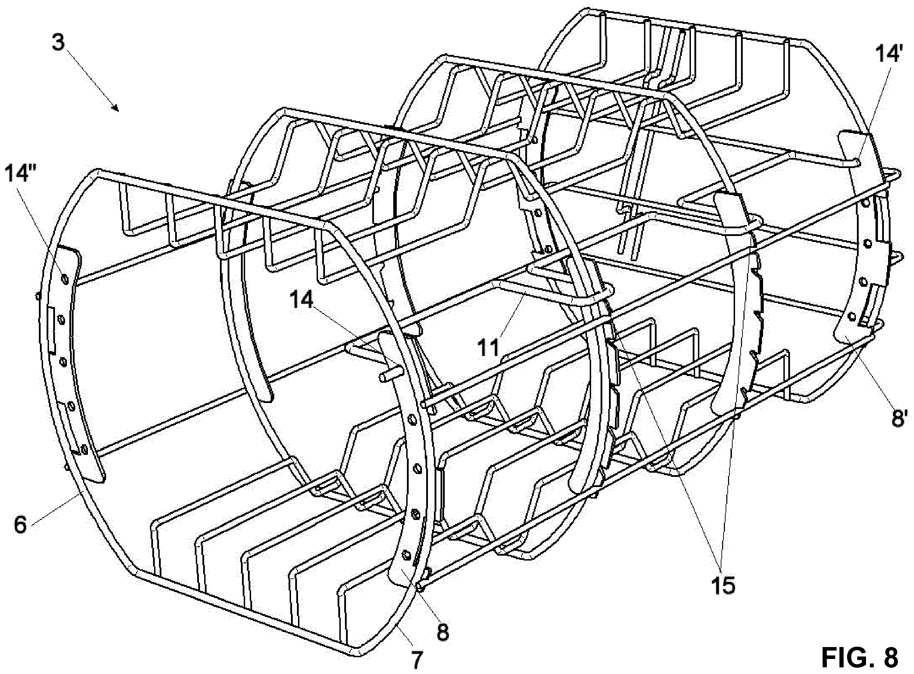

- Insertion of a second end of the same removable bracket 11 in a second hole 14 obtained in bracket support 8 fixed to a second extremity ring 20 on a second curved side 7; said holes must be at the same height (Figure 7);
- Blocking said removable bracket 11 in its working position, thanks to the presence of recesses 11 obtained in the outside side of bracket blocks 9 (Figure 8).

Obviously the same operation must be repeated with a second removable bracket 11 on the opposed curved side 6, in order to obtain two supports aligned at the same height supporting a single sterilization cassette 13 so that it lies on a plane (parallel to the floor of said autoclave 1).

In its preferred embodiment, said modular tray support 3 is made of a metallic material (AISI steel, electrochemically polished on its surface, preferably AISI steel 304), resistant to the typical corrosion and high temperatures of an autoclave (about 140°C). For bracket assembly the elastic component of the material is exploited, allowing flexion and not permanent distortion. The position of removable bracket 11 is maintained thanks to the elastic component of the material of the bracket itself, and by recesses 15 on bracket blocks 9.

Each removable bracket 11 is fixable in a wholly independent way from the others, and this allows obtaining a plurality of different configurations of use of sterilization chamber 2.

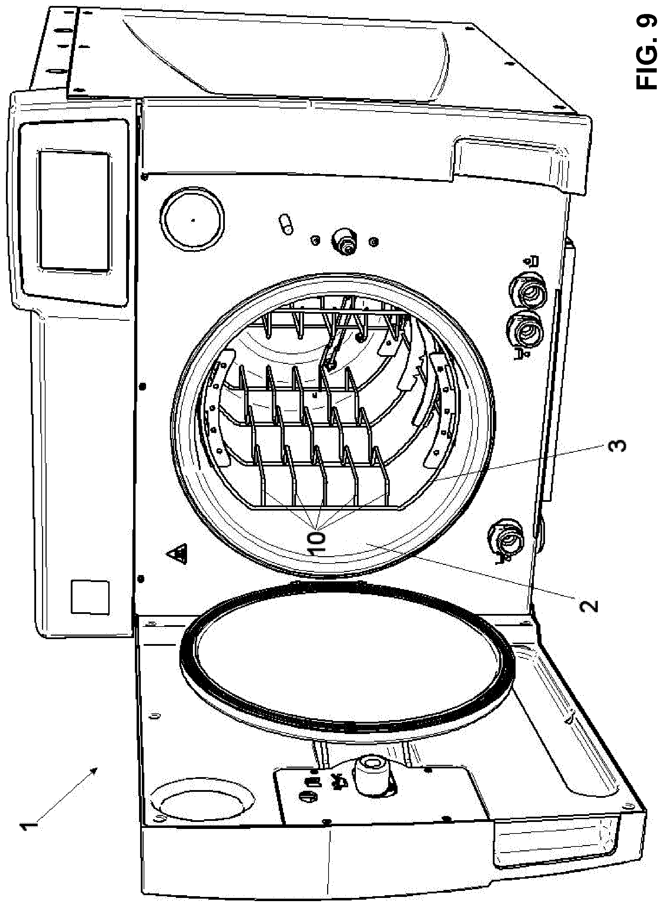

As shown in Figure 9, a further configuration for using modular tray support 3 consist in using its configuration free from removable brackets 11, turning 90 degrees said tray support 3, i.e. bringing its planar sides 4, 5 perpendicular with respect to autoclave floor. The fixed longitudinal elements 10 are then used as supports for the trays provided with said autoclave as standard kit, which are apparently of known dimensions.

Wholly indicatively, the diameter of a sterilization chamber 2 ranges 20-40 cm, while its depth ranges 30-50 cm. Always indicatively, the distance between two adjacent holes is 20-21 mm. Consequently, e.g. assembling two removable brackets 11 skipping an intermediate hole 14, a sterilization cassette 13 thick up to 4 cm can be loaded, and skipping two intermediate holes 14 a sterilization cassette 13 thick up to 6 cm can be loaded.

In the same way, cassettes having small thicknesses can be loaded directly on longitudinal fixed elements 10, while it is advantageous loading cassettes having wider width using the brackets fixed to curved sides in their larger diameter.

In a further, not shown, embodiment, removable brackets 11 are provided having different shapes, in particular in the length of the protrusions toward the lumen of said sterilization camber 2. This can allow, e.g., to support sterilization cassettes having a great thickness but a small width.

According to a further embodiment, fixed longitudinal elements 10 and/or removable brackets 11 are made of a metallic wire, which is opportunely shaped so as to have at least a bi-dimensional development in a pre-set plane. The shaping of said metallic wire forms a plurality of successive snares toward the inside of said rings. Removable brackets 11 have one or more snares external to said rings, which are intended to engage in recesses 15. In fixed longitudinal elements 10, internal snares are interposed with snares extending in the opposed direction, forming fixing branches for fixing to each ring. Rings 20, 21 can be realized through metallic wire, too.

Concerning the manufacture of extremity rings 20 and intermediate rings 21, their curved sides 6, 7 must be produced through calendering, which is a relatively expensive process.

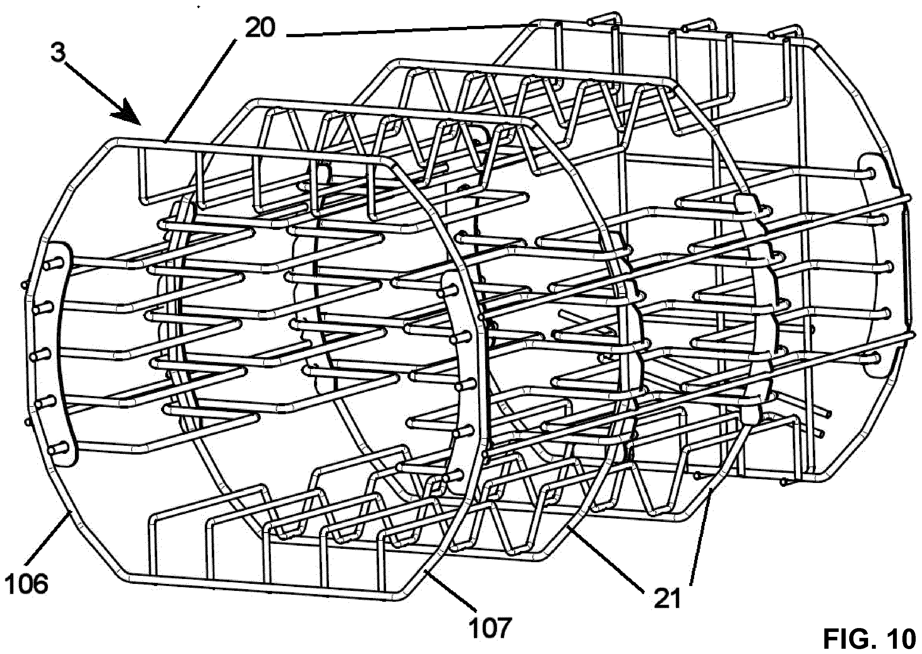

In an alternative, cheaper to produce embodiment, said curved sides 6, 7 of rings 20, 21 are manufactured through simple bending. Geometrically it is known that an arc can be approximated to a simple polygonal chain composed by a limited number of line segments with their vertices placed at regular intervals along the arc itself. When the length of line segments tends to zero, the length and trend of said polygonal chain tends to said arc. In the preferred embodiment shown in Figure 10, the curved sides 6, 7 of rings 20, 21 were replaced with two simple polygonal chains 106, 107 provided each with seven line segments. It is apparent that the number of line segments can be freely varied.
- 1: Autoclave
- 2: Sterilization chamber
- 3: Modular tray support
- 4: Planar side
- 5: Planar side
- 6: Curved side
- 7: Curved side
- 8: Bracket support
- 9: Bracket block
- 10: Fixed longitudinal element
- 11: Removable bracket
- 12: Sterilization tray
- 13: Sterilization cassette
- 14: Hole
- 15: Recess
- 20: Extremity ring
- 21: Intermediate ring
- 106: Polygonal side
- 107: Polygonal side

## Claims

1. Modular tray support (3), positionable in a sterilization chamber (2) of an autoclave (1) for sterilizing surgical/dental/veterinary instruments, for supporting sterilization trays (12) and/or cassettes (13) containing said instruments to be sterilized, wherein said tray support has a tubular structure comprising:
- at least three identical parallel frames: two extremity frames (20, 20'), and at least an intermediate frame (21);
- each of said at least three frames having two opposite lateral sides (6, 7) and at least one upper and one bottom side (4, 5)
- said frames being kept at a distance one from the other by at least two fixed longitudinal elements (10), at least one element (10) being along the upper sides (4) of the frames and at least one element (10) being provided along the bottom sides (5) of the frames;
- wherein at least one bracket (11) for supporting sterilization trays/cassettes (12, 13) is releasably mechanically coupled to each of two opposite lateral sides (6, 7) of the frames, the said brackets being provided with a zig-zag shape
**characterized in that**
- two brackets supports (8, 8') are fixed on the opposite lateral sides (6, 7) of each extremity frame (20, 20'), said bracket supports (8, 8') provided with holes (14, 14') for inserting the ends of said removable brackets (11), while each intermediate frame (21) is provided with brackets blocks (9) fixed externally to the lateral sides (6, 7) of said intermediate frame (21), said bracket blocks (9) provided with recesses (15), for blocking said removable brackets (11).

2. Modular tray support (3) for an autoclave (1) for sterilizing instruments according to claim 1, in which the longitudinal elements (10) have a zigzag shape and the upper and bottom sides (4, 5) of each of the at least three frames are planar and the lateral sides (6, 7) of each of the at least three frames are curved in the form of arcs or polygonal chains.

3. Modular tray support (3) for an autoclave (1) for sterilizing instruments according to one of the preceding claims 1 to 2, wherein said fixed longitudinal elements (10) have a zigzag shape suitable to partially protrude toward the lumen of said sterilization chamber (2), said elements being suitable to use as supports for said sterilization trays (12).

4. Autoclave (1) for sterilizing surgical/dental/veterinary instruments, comprising a sterilization chamber (2) inside which sterilization trays/cassettes (12, 13) containing said instruments are loadable,
**characterized in that** inside said sterilization chamber (2) there is provided a modular tray support (3) according to one of claims 1-3, adaptable to the dimensions of said trays/cassettes (12, 13).

5. Method for assembling a modular tray support (3) according to claim 2 or claim 3, **characterized in that** it comprises the following steps:
a. Optional extraction of said modular tray support (3) from said sterilization chamber (2);
b. Insertion of a first end of a removable bracket (11) in a first hole (14') of the bracket support (8') fixed to the lateral side (6) of the first extremity frame (20');
c. Insertion of the second extremity of the same removable bracket (11) in a second hole (14) in the bracket support (8) fixed to said second extremity frame, said two holes (14, 14') being at the same height with respect to the ground;
d. Blocking said removable bracket (11) in its working position, thanks to the presence of recesses (15) on the bracket blocks (9) fixed to intermediate frames (21);
e. Optional insertion of said modular tray support (3) into said sterilization chamber (2),
wherein said steps b, c, d are repeated with a second removable bracket (11) on the opposed lateral side (6) of the extremity frames inserting it in corresponding holes (14, 14') at the same height with respect to the ground, so that said tray/cassette (12,13) once leant on said two removable brackets (11) lays on a plane parallel to ground.

6. Method for assembling a modular tray support (3) according to claim 5, wherein all removable brackets (11) are disassembled, said modular tray support (3) is inserted rotated 90 degrees into said sterilization chamber (2), while fixed longitudinal elements (10) support trays/cassettes (12, 13) so that said tray/cassette (12,13) once leant on said longitudinal elements (10) lays on a plane parallel to the ground.

## Patentansprüche

1. Modularer Tablettträger (3), der in einer Sterilisationskammer (2) eines Autoklavs (1) zum Sterilisieren von chirurgischen/zahnärztlichen/veterinärmedizinischen Instrumenten positionierbar und zum Tragen von Sterilisationstabletts (12) und/oder -kassetten (13), die zu sterilisierenden Instrumente enthalten, vorgesehen ist, wobei der Tablettträger eine Rohrstruktur aufweist, umfassend:
- mindestens drei gleiche parallele Gestelle: zwei Endgestelle (20, 20') und mindestens ein Zwischengestell (21);
- jedes der mindestens drei Gestelle zwei gegenüberliegende Lateralseiten (6, 7) und mindestens eine Ober- und eine Unterseite (4, 5) aufweist,
- wobei die Gestelle durch mindestens zwei feste Längselemente (10) voneinander beabstandet gehalten sind und zumindest ein Element (10) entlang der Oberseiten (4) der Gestelle und zumindest ein Element (10) entlang der Unterseiten (5) der Gestelle vorgesehen sind;
- wobei mindestens eine Konsole (11) zur Abstützung von Sterilisationstabletts/-kassetten (12, 13) an zwei gegenüberliegenden Lateralseiten (6, 7) der Gestelle lösbar mechanisch gekoppelt ist, wobei die Konsolen zickzackförmig ausgebildet sind
**dadurch gekennzeichnet, dass**
- an den gegenüberliegenden Lateralseiten (6, 7) jedes Endgestells (20, 20') zwei Konsolen-Träger (8, 8') befestigt sind, wobei die Konsolen-Träger (8, 8') mit Löchern (14, 14') zum Einsetzen der Enden der abnehmbaren Konsolen (11) versehen sind, während jedes Zwischengestell (21) mit außen an den Lateralseiten (6, 7) des Zwischengestells (21) befestigten Konsolenblöcken (9) versehen ist, welche Konsolenblöcke (9) mit Ausnehmungen (15) zum Blockieren der abnehmbaren Konsolen (11) versehen sind.

2. Modularer Tablettträger (3) für einen Autoklav (1) zum Sterilisieren von Instrumenten nach Anspruch 1, bei der die Längselemente (10) zickzackförmig ausgebildet sind und die Ober- und Unterseiten (4, 5) jedes der mindestens drei Gestelle eben sind und die Lateralseiten (6, 7) jedes der mindestens drei Gestelle in Form von Bögen oder Polygonketten gekrümmt sind.

3. Modularer Tablettträger (3) für einen Autoklav (1) zum Sterilisieren von Instrumenten nach einem der vorhergehenden Ansprüche 1 bis 2, wobei die festen Längselemente (10) eine derart zickzackartige Form aufweisen, die teilweise in Richtung auf das Lumen der Sterilisationskammer (2) ragt, wobei die Elemente als Träger für die Sterilisation von Tabletts (12) dienen.

4. Autoklav (1) zum Sterilisieren von chirurgischen/zahnärztlichen/veterinärmedizinischen Instrumenten, mit einer Sterilisationskammer (2), in der diese Instrumente enthaltenen Sterilisationstabletts/-kassetten (12, 13) ladbar sind, **dadurch gekennzeichnet, dass** innerhalb der Sterilisationskammer (2) ein modularer Tablettträger (3) nach einem der Ansprüche 1 bis 3 vorgesehen ist, der an die Abmessungen der Tabletts/Kassetten (12, 13) anpassbar ist.

5. Verfahren zur Montage eines modularen Tablettträgers (3) nach Anspruch 2 oder 3, **dadurch gekennzeichnet, dass** es folgende Schritte umfasst:
a. Optionales Herausziehen des modularen Tablettträgers (3) aus der Sterilisationskammer (2);
b. Einsetzen eines ersten Endes einer abnehmbaren Konsole (11) in ein an der Lateralseite (6) des ersten Endgestells (20') befestigtes erstes Loch (14') des Konsolen-Trägers (8');
c. Einsetzen des zweiten Endes der gleichen abnehmbaren Konsole (11) in ein zweites Loch (14) in dem Konsolen-Träger (8), der an dem zweiten Endgestell befestigt ist, wobei die zwei Löcher (14, 14') sich in der gleichen Höhe relativ zum Boden befinden;
d. Blockieren der abnehmbaren Konsole (11) in ihrer Arbeitsstellung dank der Anwesenheit von Ausnehmungen (15) an den an Zwischengestellen (21) befestigten Konsolenblöcken (9);
e. Optionales Einsetzen des modularen Tablettträgers (3) in die Sterilisationskammer (2),
wobei die Schritte b, c und d auf der gegenüberliegenden Lateralseite (6) der Endgestelle mit einer zweiten abnehmbaren Konsole (11) wiederholt werden, die in entsprechende Löcher (14, 14') in gleichen Höhe relativ zum Boden derart eingeführt wird, dass das Tablett/die Kassette (12,13), nachdem es/sie auf den beiden abnehmbaren Konsolen (11) aufliegt, sich in einer Ebene parallel zum Boden befindet.

6. Verfahren zur Montage eines modularen Tablettträgers (3) nach Anspruch 5, wobei alle abnehmbare Konsolen (11) demontiert sind, der modulare Tablettträger (3) um 90 Grad gedreht in die Sterilisationskammer (2) eingeführt wird, während feste Längselemente (10) die Tabletts/Kassetten (12, 13) derart tragen, dass das Tablett/ die Kassette (12,13) nachdem es/sie auf den Längselementen (10) aufliegt, sich in einer Ebene parallel zum Boden befindet.

## Revendications

1. Support de plateau modulaire (3), pouvant être positionné dans une chambre de stérilisation (2) d'un autoclave (1) pour stériliser des outils chirurgicaux/dentaires/vétérinaires pour supporter des plateaux de stérilisation (12) et/ou des casiers (13) contenant lesdits outils à stériliser, dans lequel ledit support de plateau a une structure tubulaire comprenant:
- au moins trois structures parallèles identiques: deux structures d'extrémité (20, 20') et au moins une structure intermédiaire (21);
- chacune desdites au moins trois structures ayant deux côtés latéraux opposés (6, 7) et au moins un côté supérieur et un côté inférieur (4, 5)
- lesdites structures étant maintenues à distance les unes des autres par au moins deux éléments longitudinaux fixes (10), au moins un élément (10) étant le long des côtés supérieurs (4) des structures et au moins un élément (10) étant prévu le long des côtés inférieurs (5) des structures;
- dans lequel au moins une tablette (11) pour supporter les plateaux/casiers de stérilisation (12, 13) est couplé mécaniquement de manière amovible à chacun des deux côtés latéraux opposés (6, 7) des structures, lesdites tablettes ayant une forme en zigzag
**caractérisé en ce que**
- deux supports des tablettes (8, 8') sont fixés sur les côtés latéraux opposés (6, 7) de chaque structure d'extrémité (20, 20'), lesdits supports des tablettes (8, 8') étant pourvus de trous (14, 14') pour insérer les extrémités desdites tablettes amovibles (11), tandis que chaque structure intermédiaire (21) est pourvue de blocages (9) des tablettes fixés extérieurement aux côtés latéraux (6, 7) de ladite structure intermédiaire (21), lesdits blocages (9) des tablettes étant pourvus d'évidements (15) pour bloquer lesdites tablettes amovibles (11).

2. Support de plateau modulaire (3) pour un autoclave (1) pour stériliser des outils selon la revendication 1, dans lequel les éléments longitudinaux (10) sont en forme de zigzag et les côtés supérieurs et inférieurs (4, 5) de chacune des au moins trois structures sont plats et les côtés latéraux (6, 7) de chacune des au moins trois structures sont courbes sous forme d'arcs ou de chaînes polygonales.

3. Support de plateau modulaire (3) pour un autoclave (1) pour stériliser des outils selon l'une des revendications précédentes 1 à 2, dans lequel lesdits éléments longitudinaux fixes (10) ont une forme en zigzag appropriée pour faire saillie partiellement vers le passage de ladite chambre de stérilisation (2), lesdits éléments étant appropriés pour être utilisés comme supports pour lesdits plateaux de stérilisation (12).

4. Autoclave (1) pour stériliser des outils chirurgicaux/dentaires/vétérinaires, comprenant une chambre de stérilisation (2) à l'intérieur de laquelle des plateaux/casiers (12, 13) contenant lesdits outils peuvent être chargées,
**caractérisé en ce qu'**un support de plateau modulaire (3) selon l'une des revendications 1-3, pouvant être adapté aux dimensions desdits plateaux/casiers (12, 13), est prévu à l'intérieur de ladite chambre de stérilisation (2).

5. Méthode pour assembler un support de plateau modulaire (3) selon la revendication 2 ou la revendication 3, **caractérisé en ce qu'**elle comprend les étapes suivantes:
a. Extraction optionnelle dudit support de plateau modulaire (3) de ladite chambre de stérilisation (2);
b. Insertion d'une première extrémité d'une tablette amovible (11) dans un premier trou (14') du support de la tablette (8') fixé au côté latéral (6) de la première structure d'extrémité (20');
c. Insertion d'une seconde extrémité du même tablette amovible (11) dans un second trou (14) à l'intérieur du support de la tablette (8) fixé à ladite seconde structure d'extrémité, lesdits deux trous (14, 14') étant à la même hauteur par rapport au sol;
d. Bloquer ladite tablette amovible (11) dans sa position de travail, grâce à la présence d'évidements (15) sur les blocages (9) de la tablette fixés aux structures intermédiaires (21);
e. Insertion optionnelle dudit support de plateau modulaire (3) à l'intérieur de ladite chambre de stérilisation (2),
dans laquelle lesdites étapes b, c, d sont répétées avec une seconde tablette amovible (11) sur le côté latéral opposé (6) des structures d'extrémité en l'insérant dans des trous correspondants (14, 14') à la même hauteur par rapport au sol, afin que ledit plateau/casier (12, 13) repose sur un plan parallèle au sol une fois appuyé sur les deux tablettes amovibles (11).

6. Méthode pour assembler un support de plateau modulaire (3) selon la revendication 5, dans laquelle tous les tablettes amovibles (11) sont démontés, ledit support de plateau modulaire (3) est inséré tourné de 90 degrés à l'intérieur de ladite chambre de stérilisation (2), tandis que des éléments longitudinaux fixes (10) supportent les plateaux/casiers (12, 13) afin que ledit plateau/casier (12, 13) repose sur un plan parallèle au sol une fois appuyé sur lesdits éléments longitudinaux (10).
